# FASCICULE DE BREVET EUROPEEN

(11) **EP 1 932 819 B1**
(45) Date de publication et mention de la délivrance du brevet: **30.09.2009**
(21) Numéro de dépôt: 07291403.9
(22) Date de dépôt: 26.11.2007
(51) Int. Cl.: C07C 5/27, B01J 29/74

(54) **Procédé d'isomérisation des composés C8 aromatiques en présence d'un catalyseur comprenant une zéolithe EUO modifiée**
Isomerisationsverfahren von aromatischen C8-Verbindungen in Gegenwart eines Katalysators, der einen modifizierten Zeolith (Typ EUO) umfasst
Isomerisation method for aromatic C8 compounds in the presence of a catalyst including a modified EUO zeolite

(30) Priorité: 13.12.2006 FR 0610979
(43) Date de publication de la demande: 18.06.2008
(73) Titulaire: IFP, 92852 Rueil-Malmaison Cédex (FR)
(72) Inventeur: Gnep, Ngi Suor, 86800 Bignoux (FR); Guillon, Emmanuelle, 693390 Vernaison (FR); Lacombe, Sylvie, 69390 Vernaison (FR); Simon, Laurent, 69100 Villeurbanne (FR); Moreau, Pierre, 19200 Ussel (FR); Magnoux, Patrick, 86000 Poitiers (FR)

(56) Documents cités:
- US-A- 4 695 667
- US-A- 6 051 519
- US-A- 6 057 486
- US-A1- 2005 000 859
- US-B1- 6 660 896

## Description

La présente invention concerne l'isomérisation d'une charge aromatique comprenant au moins un composé aromatique à huit atomes de carbone. Ladite charge visée dans la présente invention est une charge contenant un mélange de xylènes, de l'éthylbenzène ou un mélange de xylènes et d'éthylbenzène. Cette charge est communément appelée "coupe C8 aromatique".
La présente invention concerne plus particulièrement un procédé d'isomérisation d'une charge aromatique comprenant au moins un composé aromatique à huit atomes de carbone par molécule visant à maximiser la production de para-xylène.

### Etat de la technique antérieure

La catalyse de l'isomérisation de l'éthylbenzène en xylènes nécessite la présence d'un métal du groupe VIII. Les formulations optimisées à base de mordénite et d'un métal du groupe VIII conduisent à des catalyseurs avec lesquels les réactions parasites restent non négligeables. Par exemple, on peut citer l'ouverture de cycles naphténiques, suivie ou non de craquage, ou encore les réactions de dismutation et de transalkylation des aromatiques en C8, qui conduisent à la formation d'aromatiques non recherchés. Il est donc particulièrement intéressant de trouver de nouveaux catalyseurs plus sélectifs.
Parmi les zéolithes utilisées en isomérisation d'une coupe C8 aromatique, on trouve la ZSM-5, utilisée seule ou en mélange avec d'autres zéolithes, comme par exemple la mordénite. Ces catalyseurs sont notamment décrits dans les brevets US-A-4 467 129, US-A-4 482 773 et EP-B-13 617. D'autres catalyseurs principalement à base de mordénite ont été décrits par exemple dans la demande de brevet FR-A-2 477 903 et dans les brevets US-A-4 723 051 et US-A-4 665 258. Le manque de sélectivité de la mordénite peut être atténué par une optimisation des formulations et/ou par des traitements spécifiques de désalumination, comme cela a par exemple été décrit dans le brevet FR-B-2 691 914 au nom de la demanderesse. Ces techniques permettent de diminuer les réactions parasites de dismutation.
Plus récemment, il a été proposé un catalyseur à base d'une zéolithe de type structural EUO (EP-A1-0 923 987). La demande de brevet WO-A-2005/065 380 décrit l'utilisation d'une zéolithe de type structural MTW en isomérisation des xylènes et de l'éthylbenzène.
Par ailleurs, plusieurs brevets ont déjà fait état de méthodes pour modifier des zéolithes afin d'améliorer l'activité ou la sélectivité des catalyseurs pour différents procédés catalytiques. En particulier, le brevet US 4,402,867 décrit une méthode de préparation d'un catalyseur à base de zéolithe comprenant une étape consistant à déposer en phase aqueuse au moins 0,3% poids de silice amorphe à l'intérieur des pores de la zéolithe. Le brevet US 4,996,034 décrit un procédé de substitution d'atomes d'aluminium présents dans une charpente zéolithique par des atomes de silicium, ledit procédé étant réalisé en une étape en milieu aqueux utilisant des sels de fluorosilicates. Le brevet US 4,451,572 décrit la préparation d'un catalyseur zéolithique comprenant une étape de dépôt de matières organosiliciques en phase vapeur ou liquide, les zéolithes visées étant des zéolithes à larges pores, en particulier la zéolithe Y.

### Résumé et intérêt de l'invention

La présente invention concerne un procédé d'isomérisation d'une charge comprenant au moins un composé aromatique à huit atomes de carbone par molécule réalisé en présence d'au moins un catalyseur comprenant au moins une zéolithe modifiée de type structural EUO, au moins un liant et au moins un métal du groupe VIII de la classification périodique des éléments, ledit catalyseur ayant été préparé selon un procédé comportant successivement au moins :
a) une étape de traitement d'une zéolithe de type structural EUO en présence d'au moins un composé moléculaire contenant au moins un atome de silicium, au cours de laquelle ledit composé, présentant un diamètre supérieur au diamètre d'ouverture maximal des pores de ladite zéolithe, est déposé sur la surface externe de ladite zéolithe en phase gazeuse,
b) au moins une étape de traitement thermique,
c) la mise en forme de ladite zéolithe avec un liant,
d) au moins une étape d'introduction d'au moins un métal du groupe VIII de la classification périodique des éléments sur un support à base de ladite zéolithe modifiée et mise en forme.

Ledit catalyseur utilisé pour la mise en oeuvre du procédé d'isomérisation selon l'invention se présente sous forme d'extrudés ou de billes. Il comprend avantageusement au moins un métal additionnel choisi dans le groupe formé par les éléments des groupes IIIA, IVA et VIIB de la classification périodique des éléments.

Il a été découvert de façon surprenante qu'un catalyseur sous forme d'extrudés ou de billes comprenant au moins une zéolithe de type structural EUO et modifiée de telle sorte qu'une couche de silice amorphe soit déposée sur la surface externe des cristaux de ladite zéolithe, au moins un liant et au moins un métal du groupe VIII de la classification périodique des éléments conduit à des performances catalytiques améliorées en terme d'activité lorsqu'il est utilisé dans un procédé d'isomérisation d'une charge comprenant au moins un composé aromatique à huit atomes de carbone par molécule. En particulier, un tel catalyseur est plus actif qu'un catalyseur à base d'une zéolithe de type structural EUO modifiée par une étape de traitement réalisée en phase liquide en présence d'au moins un composé moléculaire comprenant au moins un atome de silicium. Il est également particulièrement sélectif envers les produits recherchés, à savoir les xylènes et en particulier le para-xylène. Il est particulièrement avantageux de disposer d'un catalyseur à la fois actif et sélectif, ce qui permet d'augmenter le rendement en produit désiré. Une couche de silice déposée sur la surface externe via un traitement en phase gazeuse permet d'obtenir une haute activité et haute sélectivité.

### Descritption de l'invention

La présente invention porte sur un procédé d'isomérisation d'une charge comprenant au moins un composé aromatique à huit atomes de carbone par molécule réalisé en présence d'au moins un catalyseur comprenant au moins une zéolithe modifiée de type structural EUO, au moins un liant et au moins un métal du groupe VIII de la classification périodique des éléments, ledit catalyseur ayant été préparé selon un procédé comportant successivement au moins :
a) une étape de traitement d'une zéolithe de type structural EUO en présence d'au moins un composé moléculaire contenant au moins un atome de silicium, au cours de laquelle ledit composé, présentant un diamètre supérieur au diamètre d'ouverture maximal des pores de ladite zéolithe, est déposé sur la surface externe de ladite zéolithe en phase gazeuse,
b) au moins une étape de traitement thermique,
c) la mise en forme de ladite zéolithe avec un liant,
d) au moins une étape d'introduction d'au moins un métal du groupe VIII de la classification périodique des éléments sur un support à base de ladite zéolithe modifiée et mise en forme.

Conformément à l'invention, ledit catalyseur utilisé pour la mise en oeuvre du procédé d'isomérisation selon l'invention comprend au moins un métal du groupe VIII choisi parmi le fer, le cobalt, le nickel, le ruthénium, le rhodium, le palladium, l'osmium, l'iridium et le platine, de préférence choisi parmi les métaux nobles, de manière très préférée choisi parmi le palladium et le platine et de manière encore plus préférée le platine. La dispersion du(es) métal(ux) du groupe VIII, déterminée par chimisortpion, par exemple par titration H₂-O₂ ou par chimisorption du monoxyde de carbone, est comprise entre 50% et 100%, de préférence entre 60 % et 100% et de manière encore plus préférée entre 70% et 100%. Le coefficient de répartition macroscopique du(es) métal(ux) du groupe VIII, obtenu à partir de son profil déterminé par microsonde de Castaing, défini comme le rapport des concentrations du(es) métal(ux) du groupe VIII au coeur du grain par rapport au bord de ce même grain, est compris entre 0,7 et 1,3, de préférence entre 0,8 et 1,2. La valeur de ce rapport, voisine de 1, témoigne de l'homogénéité de la répartition du(es) métal(ux) du groupe VIII dans le catalyseur.

Ledit catalyseur comprend avantageusement au moins un métal additionnel choisi dans le groupe formé par les éléments des groupes IIIA, IVA et VIIB de la classification périodique des éléments et de préférence choisi parmi le gallium, l'indium, l'étain et le rhénium. Ledit métal additionnel est de préférence choisi parmi l'indium, l'étain et le rhénium.

Ledit catalyseur comprend également avantageusement du soufre.

Ledit catalyseur utilisé pour la mise en oeuvre du procédé d'isomérisation selon l'invention contient plus particulièrement :
- de 1 à 90%, de préférence de 3 à 80% et de manière encore plus préférée de 4 à 60% poids de ladite zéolithe de type structural EUO modifiée,
- de 0,01 à 4%, de préférence de 0,05 à 2,0% poids d'au moins un métal du groupe VIII de la classification périodique des éléments,
- éventuellement de 0,01 à 2%, de préférence de 0,05 à 1% poids d'au moins un métal additionnel choisi dans le groupe formé par les éléments des groupes IIIA, IVA et VIIB,
- éventuellement une teneur en soufre telle que le rapport du nombre d'atomes de soufre sur le nombre d'atome de(s) métal(ux) du groupe VIII soit compris entre 0,3:1 et 2:1,
- au moins un liant assurant le complément à 100% dans le catalyseur.

Ledit catalyseur se présente sous forme de billes ou d'extrudés, de préférence sous forme d'extrudés.

Conformément à l'invention, la zéolithe initiale de type structural EUO, n'ayant pas encore été modifiée pour être comprise dans le catalyseur utilisé pour la mise en oeuvre du procédé d'isomérisation selon l'invention, présente un réseau microporeux monodimensionnel, dont le diamètre des pores est de 4,1 x 5,4 Å (1 Å = 1 Angström = 10⁻¹⁰ m) (« Atlas of Zeolite Framework Types », Ch. Baerlocher, W.M. Meier et D.H. Olson, 5ème Edition, 2001). D'autre part, N.A. Briscoe *et al* ont enseigné dans un article de la revue Zeolites (1988, 8, 74) que ces canaux monodimensionnels possèdent des poches latérales de profondeur 8,1 Å et de diamètre 6,8 x 5,8 Å. Ladite zéolithe de type structural EUO est choisie parmi les zéolithes EU-1, ZSM-50 et TPZ-3, de préférence il s'agit de la zéolithe EU-1.
Le mode de synthèse de la zéolithe EU-1 et ses caractéristiques physico-chimiques ont déjà été décrits dans le brevet EP-B1- 42 226. Le brevet US-4.640.829 décrit la zéolithe ZSM-50 et son procédé de préparation. La zéolithe TPZ-3 et son procédé de préparation sont divulgués dans la demande de brevet EP-A1-51 318.
La zéolithe de type structural EUO, modifiée selon les différentes étapes du procédé de préparation du catalyseur utilisé pour la mise en oeuvre du procédé d'isomérisation selon l'invention, contient initialement, c'est-à-dire avant d'être modifiée selon ladite étape a), au moins du silicium et au moins un élément T choisi dans le groupe formé par l'aluminium, le fer, le gallium et le bore, de préférence l'aluminium et le bore et de manière très préférée l'aluminium dans une proportion telle que le rapport atomique Si/T, de préférence le rapport atomique Si/AI, est de préférence compris entre 2:1 et 100:1, de manière plus préférée compris entre 5:1 et 80:1 et de manière encore plus préférée compris entre 5:1 et 50:1. Ladite zéolithe de type structural EUO, de préférence ladite zéolithe EU-1, employée pour la mise en oeuvre de ladite étape a) du procédé de préparation du catalyseur utilisé pour la mise en oeuvre du procédé d'isomérisation selon l'invention, se présente sous forme calcinée et contient au moins un proton de telle sorte qu'elle se trouve sous sa forme protonée (forme hydrogène H⁺) dans laquelle la teneur en cation autre que H⁺ est inférieure à 30% du nombre total de cations, de préférence inférieure à 20 % et de manière très préférée inférieure à 15 % par rapport au nombre total de cations sur la zéolithe. Dans le cas où, préalablement à la mise en oeuvre de ladite étape a) du procédé de préparation du catalyseur, la zéolithe à modifier se trouve sous sa forme brute de synthèse, contenant encore le structurant organique utilisé pour la préparer, on pourra procéder à une calcination de ladite zéolithe à une température comprise entre 300 et 700°C, de préférence entre 400 et 600°C puis si la zéolithe contient un ou plusieurs métal(aux) alcalin(s)/alcalino-terreux, on procédera à un ou plusieurs échange(s) ionique(s) par une solution contenant au moins un sel d'ammonium, par exemple le nitrate d'ammonium NH₄NO₃, de manière à éliminer au moins en partie, de préférence pratiquement totalement, un cation alcalin présent dans la zéolithe. Une étape de calcination sous flux d'air sec, à une température généralement comprise entre environ 400 et 500°C, a ensuite pour but de générer la formation des protons dans la zéolithe par désorption d'ammoniaque conduisant ainsi à la forme hydrogène de la zéolithe, prête pour la mise en oeuvre de ladite étape a) du procédé de préparation du catalyseur utilisé pour la mise en oeuvre du procédé d'isomérisation selon l'invention.

Ladite zéolithe de type structural EUO, de préférence la zéolithe EU-1, employée pour la mise en oeuvre de ladite étape a) du procédé de préparation du catalyseur, est une zéolithe acide contenant entre 70 et 100%, de préférence entre 80 et 100% et de manière très préférée entre 85 et 100% de cations de compensation de forme protonique H⁺, le reste des cations étant choisi de manière préférée parmi les métaux des groupes lA et IIA de la classification périodique des éléments, et plus particulièrement le(s)dit(s) cation(s) est(sont) choisi(s) parmi les cations Na⁺, Li⁺, K⁺, Rb⁺, Cs⁺, Ba²⁺ et Ca²⁺. En particulier, la teneur en sodium est de préférence telle que le rapport atomique Na/T, de préférence le rapport Na/Al, est inférieur à 0,5:1, de préférence inférieur à 0,1:1, de manière encore plus préférée inférieur à 0,02:1.

La zéolithe de type structural EUO, par exemple la zéolithe EU-1, entrant dans la composition du catalyseur utilisé pour la mise en oeuvre du procédé d'isomérisation selon l'invention présente généralement une surface spécifique, mesurée par chimisorption d'azote selon la méthode BET, supérieure à 300 m²/g. De préférence, sa surface spécifique est supérieure à 400 m²/g.

Le catalyseur utilisé pour la mise en oeuvre du procédé d'isomérisation selon l'invention est préparé selon un procédé comportant, dans un premier temps, au moins une étape a) consistant en la sélectivation de ladite zéolithe de type structural EUO, de préférence de la zéolithe EU-1. Par "sélectivation", on entend au sens de la présente invention, la neutralisation de l'acidité de la surface externe de chacun des cristaux de la zéolithe. La neutralisation de l'acidité peut se faire par toute méthode connue de l'Homme du métier. Les méthodes conventionnelles emploient généralement, pour réaliser la sélectivation spécifique des sites acides de la surface externe d'une zéolithe, des molécules dont le diamètre cinétique est supérieur au diamètre de l'ouverture des pores de la zéolithe. Plus précisément, ladite étape a) de sélectivation consiste à traiter ladite zéolithe de type structural EUO, se présentant sous sa forme protonée, en présence d'au moins un composé moléculaire contenant au moins un atome de silicium dont le diamètre est supérieur au diamètre d'ouverture maximal des pores de la zéolithe de type structural EUO, de préférence de la zéolithe EU-1, à traiter selon ladite étape a). De préférence, le procédé de préparation du catalyseur ne comprend qu'une seule étape a).

Les molécules généralement utilisées pour passiver ou sélectiver la surface externe de la zéolithe de type structural EUO, de préférence la zéolithe EU-1, sont des composés contenant des atomes pouvant interagir avec les sites de surface externe de chacun des cristaux de la zéolithe. Les molécules utilisées selon l'invention sont des molécules organiques ou inorganiques contenant un ou plusieurs atome(s) de silicium. Aussi, conformément à ladite étape a) de traitement du procédé de préparation du catalyseur utilisé pour la mise en oeuvre du procédé d'isomérisation selon l'invention, la zéolithe protonée est soumise à une étape de traitement en présence d'au moins un composé moléculaire contenant au moins un atome de silicium. Ladite étape a) permet le dépôt d'une couche dudit composé moléculaire contenant au moins un atome de silicium à la surface externe de ladite zéolithe de type structural EUO qui se transformera après l'étape b) en une couche de silice amorphe sur la surface externe de chacun des cristaux de la zéolithe. De manière préférée, le composé moléculaire contenant au moins un atome de silicium est choisi parmi les composés de formule Si-R₄ et Si₂-R₆ où R peut être soit de l'hydrogène, soit un groupe alkyle, aryle ou acyle, soit un groupe alkoxy (O-R'), soit un groupe hydroxyl (-OH) soit encore un halogène, de préférence un groupe alkoxy (O-R'). Au sein d'une même molécule Si-R₄ ou Si₂-R₆, le groupement R peut être soit identique soit différent. Par exemple, on pourra d'après les formules décrites ci-dessus choisir des composés moléculaires de formule Si₂H₆ ou Si(C₂H₅)₃(CH₃). Ainsi, le composé moléculaire contenant au moins un atome de silicium employé dans l'étape a) du procédé de préparation du catalyseur utilisé pour la mise en oeuvre du procédé d'isomérisation selon l'invention peut être un composé de type silane, disilane, alkylsilane, alkoxysilane ou siloxane. De manière très préférée, ledit composé moléculaire présente une composition de formule générale Si-(OR')₄ où R' est un groupement alkyle, aryle ou acyle, de préférence un groupement alkyle et de manière très préférée un groupement éthyle. Ledit composé moléculaire employé pour la mise en oeuvre de ladite étape a) présente un diamètre supérieur au diamètre d'ouverture maximal des pores de la zéolithe de type structural EUO, de préférence de la zéolithe EU-1, et comprend de préférence au plus deux atomes de silicium par molécule. Le composé moléculaire tétraéthylorthosilicate (TEOS) de formule Si(OCH₂CH₃)₄, qui présente un diamètre égal à 9,6 Å, est très avantageux pour la mise en oeuvre de ladite étape a) du procédé de préparation du catalyseur utilisé pour la mise en oeuvre du procédé d'isomérisation selon l'invention.

Ladite étape a) du procédé de préparation du catalyseur, utilisé pour la mise en oeuvre du procédé d'isomérisation selon l'invention, qui consiste à traiter la zéolithe protonée en présence d'au moins un composé moléculaire contenant au moins un atome de silicium est réalisée par dépôt dudit composé sur la surface externe de la zéolithe. Conformément à l'invention, ladite étape a) est réalisée en procédant au dépôt dudit composé moléculaire contenant au moins un atome de silicium en phase gazeuse.

L'étape a) de préparation du catalyseur utilisé pour la mise en oeuvre du procédé d'isomérisation selon l'invention est réalisée dans un réacteur à lit fixe. Préalablement à la réaction de dépôt en phase gazeuse (CVD) dans ledit réacteur à lit fixe, la zéolithe de type structural EUO, de préférence la zéolithe EU-1, est préférentiellement activée. L'activation de ladite zéolithe dans le réacteur à lit fixe est réalisée sous oxygène, sous air ou sous gaz inerte, ou sous un mélange d'air et de gaz inerte ou d'oxygène et gaz inerte. La température d'activation de la zéolithe est avantageusement comprise entre 100 et 600°C, et très avantageusement entre 300 et 550°C. Le composé moléculaire contenant au moins un atome de silicium devant être déposé sur la surface externe de chacun des cristaux de la zéolithe est envoyé dans le réacteur en phase vapeur, ledit composé moléculaire étant dilué dans un gaz vecteur qui peut être soit de hydrogène (H₂), soit de l'air, soit de l'Argon (Ar), soit de l'hélium (He), soit encore de l'azote (N₂), préférentiellement le gaz vecteur est un gaz inerte choisi parmi Ar, He et N₂. Ledit composé moléculaire contenant au moins un atome de silicium est déposé sur la surface externe de ladite zéolithe en phase vapeur, en l'absence de tout composé hydrocarboné. Pour obtenir une couche de silice amorphe de qualité optimale sur la surface externe de la zéolithe de type structural EUO, il est nécessaire de bien choisir les conditions opératoires pour le dépôt du composé moléculaire contenant au moins un atome de silicium. En particulier, la température du lit de zéolithe pendant le dépôt est préférentiellement comprise entre 10 et 300°C, et très préférentiellement comprise entre 50 et 200°C, la pression partielle, dans la phase gaz, du composé moléculaire à déposer sur la surface externe de la zéolithe est préférentiellement comprise entre 0,001 et 0,5 bar, et très préférentiellement comprise entre 0,01 et 0,2 bar, la durée du dépôt est préférentiellement comprise entre 10 minutes et 10 heures et très préférentiellement comprise entre 30 minutes et 5 heures et encore plus préférentiellement entre 1 et 3 heures.

Conformément à l'étape b) du procédé de préparation du catalyseur utilisé pour la mise en oeuvre du procédé d'isomérisation selon l'invention, le composé moléculaire contenant au moins un atome de silicium est décomposé par un traitement thermique lequel est réalisé à une température préférentiellement comprise entre 200 et 700°C, plus préférentiellement entre 300 et 500°C. Ladite étape de traitement thermique est mise en oeuvre sous air, sous oxygène, sous hydrogène, sous azote ou sous argon ou sous un mélange d'azote et d'argon. La durée de ce traitement est avantageusement comprise entre 1 et 5 heures. A l'issue dudit traitement thermique, une couche de silice amorphe est déposée sur la surface externe de chacun des cristaux de la zéolithe de type structural EUO. Conformément à l'invention, la surface interne de chacun des cristaux de la zéolithe de type structural EUO est de préférence dépourvue d'un dépôt d'une couche de silice amorphe. Le diamètre d'ouverture de pores maximal de la zéolithe modifiée, présente dans le catalyseur utilisé pour la mise en oeuvre du procédé d'isomérisation selon l'invention, est préférentiellement inchangé par rapport à celui de la zéolithe initiale encore non modifiée.

Conformément à l'étape c) du procédé de préparation du catalyseur utilisé pour la mise en oeuvre du procédé d'isomérisation selon l'invention, la zéolithe de type structural EUO issue de ladite étape b) est mélangée avec au moins un liant puis est mise en forme. La mise en forme du catalyseur est généralement telle que le catalyseur se trouve sous forme d'extrudés ou de billes, de préférence sous forme d'extrudés. Les conditions de mise en forme de la zéolithe modifiée de type structural EUO, le choix du liant, éventuellement le broyage de ladite zéolithe, le procédé de peptisation, l'ajout d'agents porogènes, le temps de malaxage, la pression d'extrusion si le catalyseur est mis sous forme d'extrudés, la vitesse et le temps de séchage sont déterminés pour chaque liant selon les règles bien connues de l'Homme du métier de manière à obtenir un catalyseur sous forme de billes ou d'extrudés, de préférence sous forme d'extrudés.
Le liant est un élément minéral poreux amorphe ou mal cristallisé de type oxyde. Il est choisi dans le groupe formé par les argiles, la magnésie, les alumines, les silices, l'oxyde de titane, l'oxyde de bore, la zircone, les phosphates d'aluminium, les phosphates de titane, les phosphates de zirconium et les silices-alumines. On peut aussi utiliser du charbon. De préférence, le liant est une alumine.
La mise en forme consiste avantageusement à malaxer la zéolithe modifiée de type structural EUO, par exemple la zéolithe modifiée EU-1, dans un gel humide de liant (obtenu généralement par mélange d'au moins un acide et d'une poudre de liant), préférentiellement l'alumine, pendant une durée nécessaire à l'obtention d'une bonne homogénéité de la pâte, par exemple pendant une dizaine de minutes, puis à passer la pâte ainsi obtenue à travers une filière pour former des extrudés, par exemple de diamètre de 0,4 à 4 mm. La mise en forme est généralement suivie d'un séchage, par exemple pendant quelques heures à environ 120°C en étuve, et d'une calcination, généralement à une température de 250°C à 600°C, par exemple pendant deux heures à environ 400°C. Ladite zéolithe modifiée de type structural EUO et mise en forme constitue le support du catalyseur utilisé pour la mise en oeuvre du procédé d'isomérisation selon l'invention.

Conformément à l'étape d) du procédé de préparation du catalyseur utilisé pour la mise en oeuvre du procédé d'isomérisation selon l'invention, au moins un métal du groupe VIII est introduit sur le support à base de ladite zéolithe de type structural EUO modifiée et mise en forme. Selon la méthode mise en oeuvre pour l'introduction dudit métal du groupe VIII, comme il sera indiqué ci-après, ledit métal du groupe VIII, de préférence le platine, est déposé de manière prépondérante soit sur la zéolithe modifiée soit sur le liant. Le(s) métal(ux) du groupe VIII de la classification périodique des éléments et éventuellement le(s) métal(ux) additionnel(s) est(sont) introduit(s) soit tous de la même façon soit par des techniques différentes. De manière préférée, au moins ledit métal du groupe VIII de la classification périodique des éléments est introduit par dépôt sélectif sur le support à base de ladite zéolithe modifiée et mise en forme avec le liant par tout procédé connu de l'homme du métier. Un tel dépôt est par exemple effectué par la technique d'imprégnation à sec, la technique d'imprégnation par excès ou par échange ionique. Le dépôt est avantageusement opéré de sorte que la dispersion dudit ou desdits métal(ux) du groupe VIII, déterminée par chimisorption, soit comprise entre 50 % et 100 %, de préférence comprise entre 60 % à 100 % et de manière encore plus préférée comprise entre 70 % et 100 % et de sorte que le coefficient de répartition macroscopique dudit ou desdits métal(ux) du groupe VIII, défini comme le rapport des concentrations de chaque métal du groupe VIII au coeur du grain par rapport au bord de ce même grain, soit compris entre 0,7:1 à 1,3:1, de préférence de 0,8:1 à 1,2:1.
Pour l'introduction des différents métaux compris dans la composition du catalyseur, tous les précurseurs desdits métaux conviennent pour le dépôt de ces éléments.
Le platine, préférentiellement choisi comme métal du groupe VIII, est généralement introduit sur la zéolithe de type structural modifiée et/ou sur le liant. Pour tout métal noble du groupe VIII, on peut avantageusement utiliser des composés ammoniaqués ou des composés tels que par exemple le chloroplatinate d'ammonium, le dichlorure de platine dicarbonyle, l'acide hexahydroxyplatinique, le chlorure de palladium ou le nitrate de palladium.
Le contrôle de certains paramètres mis en oeuvre lors du dépôt dudit(desdits) métal(ux) du groupe VIII, en particulier la nature du précurseur dudit(desdits) métal(ux) du groupe VIII utilisé(s) permet d'orienter le dépôt dudit(desdits) métal(ux) majoritairement sur le liant ou sur la zéolithe.
L'introduction du(es) métal(ux) du groupe VIII, en particulier du platine, au moyen de précurseurs ammoniaqués conduit majoritairement au dépôt dudit métal, en particulier du platine, sur la zéolithe modifiée de type structural EUO. L'introduction de tels précurseurs ammoniaqués pour la préparation du catalyseur est réalisée par échange cationique. En particulier, lorsque ledit métal du groupe VIII est le platine, le précurseur est avantageusement choisi parmi les composés ammoniaqués tels que les sels de platine (II) tétrammines de formule Pt(NH₃)₄X₂, les sels de platine (IV) hexammines de formule Pt(NH₃)₆X₄ ; les sels de platine (IV) halogénopentammines de formule (PtX(NH₃)₅)X₃ et les sels de platine N-tétrahalogénodiammines de formule PtX₄(NH₃)₂. Des composés halogénés de formule H(Pt(acac)₂X), X étant un halogène choisi dans le groupe formé par le chlore, le fluor, le brome et l'iode, X étant de préférence le chlore, et "acac" représentant le groupe acétylacétonate (de formule brute C₅H₇O₂), dérivé de l'acétylacétone, sont également avantageusement choisi comme précurseur du platine pour que le platine soit majoritairement déposé sur la zéolithe modifiée de type structural EUO par échange cationique.
L'introduction du(es) métal(ux) du groupe VIII par échange anionique conduit majoritairement au dépôt dudit(desdits) métal(ux) sur le liant. En particulier, pour introduire le platine ou le palladium, on peut mettre en oeuvre un échange anionique avec de l'acide hexachloroplatinique et/ou de l'acide hexachloropalladique, en présence d'un agent compétiteur, par exemple l'acide chlorhydrique, le dépôt étant en général suivi d'une calcination, par exemple pendant environ 2 heures à environ 400°C.
L'introduction d'au moins un métal additionnel choisi dans le groupe formé par les éléments des groupes IIIA, IVA et VIIB de la classification périodique des éléments dans le catalyseur au cours de ladite étape d) de préparation dudit catalyseur peut être réalisée selon toutes les techniques de dépôt connues de l'homme du métier et tous les précurseurs desdits métaux additionnels conviennent. En particulier, on peut ajouter le(s) métal(ux) du groupe VIII et le(s) métal(ux) additionnel(s) choisi(s) parmi les métaux des groupes IIIA, IVA et VIIB soit séparément soit simultanément au cours de ladite étape d). Le(s) métal(ux) additionnel(s) peu(ven)t être introduit(s) par l'intermédiaire de précurseurs tels que par exemple les chlorures, les bromures et les nitrates d'éléments des groupes IIIA, IVA et VIIB. Par exemple dans le cas de l'indium, on utilisera avantageusement le nitrate ou le chlorure et dans le cas du rhénium, l'acide perrhénique. Le(s) métal(ux) additionnel(s) peu(ven)t également être avantageusement introduit(s) sous la forme d'au moins un composé organique choisi dans le groupe constitué par les complexes dudit(desdits) métal(ux), en particulier les complexes polycétoniques du métal et les hydrocarbylmétaux tels que les alkyles, les cycloalkyles, les aryles, les alkylaryles et les arylalkyles métaux. Dans ce dernier cas, l'introduction dudit métal additionnel est avantageusement effectuée à l'aide d'une solution du composé organométallique dudit métal dans un solvant organique. On peut également employer comme précurseur(s) du(es) métal(ux) additionnel(s) des composés organohalogénés dudit métal. On peut également avantageusement employer comme précurseur(s) du(es) métal(ux) additionnel(s) des composés organiques dudit(desdites) métal(ux) additionnel(s), en particulier le tétrabutylétain, dans le cas de l'étain, et le triphénylindium, dans le cas de l'indium.
Si le métal additionnel est introduit avant le(s) métal(ux) du groupe VIII, le précurseur dudit métal additionnel utilisé est généralement choisi dans le groupe constitué par l'halogénure, le nitrate, l'acétate, le tartrate, le carbonate et l'oxalate dudit métal additionnel. L'introduction est alors avantageusement effectuée en solution aqueuse. Il peut également être avantageux d'introduire le(s)dit(s) métal(ux) additionnel(s) à l'aide d'une solution d'un composé organométallique dudit(desdits) métal(ux), par exemple le tétrabutylétain. Dans ce cas, avant de procéder à l'introduction d'au moins un métal du groupe VIII, on procédera à une calcination sous air.
L'introduction du(es) métal(ux) du groupe VIII, de préférence le(s) métal(ux) noble(s) de la famille du platine, et éventuellement celle du métal additionnel, est de préférence effectuée par imprégnation à l'aide d'une solution aqueuse ou organique de l'un des composés organométalliques cités ci-dessus. Parmi les solvants organiques utilisables, on peut citer les hydrocarbures paraffiniques, naphténiques ou aromatiques contenant par exemple de 6 à 12 atomes de carbone par molécule, et les composés organiques halogénés contenant par exemple de 1 à 12 atomes de carbone par molécule. On peut citer par exemple le n-heptane, le méthylcyclohexane, le toluène et le chloroforme. On peut aussi utiliser les mélanges de solvants.
De plus, des traitements intermédiaires tels que par exemple une calcination et/ou une réduction peuvent être appliqués entre les dépôts successifs des différents métaux.
La préparation du catalyseur utilisé pour la mise en oeuvre du procédé d'isomérisation selon l'invention se termine généralement par une calcination, habituellement à une température d'environ 250°C à 600°C, pour une durée d'environ 0,5 à 10 heures, de préférence précédée d'un séchage, par exemple à l'étuve, à une température allant de la température ambiante à 250°C, de préférence de 40°C à 200°C. Ladite étape de séchage est de préférence menée pendant la montée en température nécessaire pour effectuer ladite calcination.
Il est avantageux de mettre en oeuvre une réduction préalable du catalyseur *ex situ,* sous courant d'hydrogène, par exemple à une température de 450°C à 600°C, pendant une durée de 0,5 à 4 heures. Dans le cas où le catalyseur utilisé pour la mise en oeuvre du procédé d'isomérisation selon l'invention ne contient pas de soufre, une réduction du(es) métal(ux) présent(s) dans la composition du catalyseur sous hydrogène est réalisée *in situ* avant injection de la charge.
Le catalyseur, utilisé pour la mise en oeuvre du procédé d'isomérisation selon l'invention, est préparé conformément aux étapes a) à d) décrites ci-dessus avant son introduction dans le réacteur réalisant la réaction d'isomérisation : le catalyseur est mis en contact avec ladite charge comprenant au moins un composé aromatique à huit atome de carbone une fois qu'au moins lesdites étapes de préparation a) à d) décrites ci-dessus sont réalisées.
Dans le cas où le catalyseur utilisé pour la mise en oeuvre du procédé d'isomérisation selon l'invention contient du soufre, le soufre est introduit sur le catalyseur mis en forme, calciné, contenant le ou les métaux cités précédemment, soit *in situ* avant la réaction catalytique, soit *ex situ.* La sulfuration éventuelle intervient après la réduction du catalyseur. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration. La sulfuration s'effectue en présence d'hydrogène en utilisant tout agent sulfurant bien connu de l'homme de métier, tel que le sulfure de diméthyle ou le sulfure d'hydrogène. Par exemple, le catalyseur est traité avec une charge contenant du sulfure de diméthyle en présence d'hydrogène, avec une concentration telle que le rapport atomique soufre/métal(ux) du groupe VIII soit compris entre 0,3:1 et 2:1. Le catalyseur est ensuite maintenu pendant environ 3 heures à environ 400°C sous débit d'hydrogène avant l'injection de la charge.
Le procédé d'isomérisation selon l'invention comprend la mise en contact d'au moins une charge comprenant au moins un composé aromatique à huit atomes de carbone par molécule avec au moins un catalyseur ayant la composition telle que décrite ci-dessus et préparé selon le procédé tel que décrit ci-dessus, ledit catalyseur étant présent dans un réacteur au sein duquel se produit la réaction d'isomérisation. Ladite charge comprenant au moins un composé aromatique à huit atomes de carbone par molécule comprend soit uniquement un mélange de xylènes soit uniquement de l'éthylbenzène soit encore un mélange de xylènes et d'éthylbenzène. Ledit procédé d'isomérisation selon l'invention est avantageusement mis en oeuvre selon les conditions opératoires suivantes :
- une température comprise entre 300°C et 500°C, de préférence entre 320°C et 450°C et de manière plus préférée entre 340°C et 430°C ;
- une pression partielle d'hydrogène comprise entre 0,3 et 1,5 MPa, de préférence entre 0,4 et 1,2 MPa et de manière plus préférée entre 0,7 et 1,2 MPa ;
- une pression totale comprise entre 0,45 et 1,9 MPa, de préférence entre 0,6 et 1,5MPa;et
- une vitesse spatiale d'alimentation, exprimée en kilogramme de charge introduite par kilogramme de catalyseur et par heure, comprise entrre 0,25 et 30 h⁻¹, de préférence entre 1 et 10 h⁻¹ et de manière plus préférée entre 2 et 6 h⁻¹.
Les exemples suivants illustrent l'invention sans toutefois en limiter la portée.

### Exemple 1 : Préparation d'un catalyseur A comprenant une zéolithe EU-1 modifiée par CVD, de l'alumine et du platine (conforme à l'invention).

La zéolithe de départ est une zéolithe EU-1 de rapport atomique Si/Al = 15, sous forme H⁺. La passivation de la surface externe est réalisée par dépôt en phase gazeuse (CVD) d'une couche de tétraéthoxysilane (TEOS).
40 g de zéolithe EU-1 sont introduits dans un réacteur à lit fixe où ils sont d'abord soumis à une activation sous flux d'azote à 550°C. La température du réacteur est ensuite ramenée à 150°C, puis une pression partielle de 0,15 bar de TEOS [Si (OCH₂CH₃)₄] est ajoutée dans le flux d'azote. Après 2 h de réaction, la zéolithe est strippée pendant 2 h sous azote à 150°C pour évacuer le TEOS n'ayant pas réagi. La décomposition du TEOS se fait ensuite sous air à 450°C pendant 3 heures.
L'analyse élémentaire montre que le rapport atomique Si/Al (mol/mol) est égal à 18 : cette augmentation du rapport atomique Si/Al s'explique par le dépôt de 17% poids par rapport au poids de la zéolithe initiale d'une couche de silice amorphe sur la surface externe de chacun des cristaux de la zéolithe EU-1.
La zéolithe EU-1 ainsi modifiée est ensuite mise en forme par extrusion (diamètre d'extrusion = 1,4 mm) avec un gel d'alumine de manière à obtenir, après séchage et calcination sous air sec, un support S1 qui contient 10 % poids de zéolithe EU-1 modifiée par CVD sous forme hydrogène et 90 % poids d'alumine.
Ce support S1 est soumis à un échange anionique avec de l'acide hexachloroplatinique en présence d'un agent compétiteur (acide chlorhydrique), de manière à déposer sur l'alumine 1 % poids de platine par rapport au poids du catalyseur. Le solide humide est ensuite séché à 120°C pendant 12 heures et calciné sous un débit d'air sec à la température de 500°C pendant une heure.
Le catalyseur A ainsi obtenu contient, en poids, 10 % de zéolithe EU-1 modifiée par CVD, 89 % d'alumine et 1 % de platine.

### Exemple 2 : Préparation d'un catalyseur B comprenant une zéolithe EU-1 modifiée par CLD, de l'alumine et du platine (non-conforme à l'invention).

La zéolithe de départ est une zéolithe EU-1 de rapport atomique Si/Al = 15, sous forme H⁺. La passivation de la surface externe est réalisée par dépôt en phase liquide (CLD) d'une couche de tétraéthoxysilane (TEOS).
40 g de zéolithe EU-1 sont introduits dans un réacteur à lit fixe où ils sont d'abord soumis à une activation sous flux d'azote à 550°C. La température du réacteur est ensuite ramenée à température ambiante. La zéolithe est ensuite mise en solution dans du toluène anhydre (V/P=10). 50g de TEOS, quantité permettant d'obtenir un matériau final avec un rapport Si/Al (mol/mol) égal à 18, sont ajoutés au toluène anhydre sous reflux. La solution est laissée à agiter pendant 2h. Après 2 h de réaction, le toluène est évaporé sous vide en utilisant un rotavapeur. La décomposition du TEOS se fait ensuite dans un réacteur en lit fixe sous air à 450°C pendant 3 heures.
L'analyse élémentaire montre que le rapport atomique Si/Al (mol/mol) est égal à 18 : cette augmentation du rapport atomique Si/Al s'explique par le dépôt de 17% poids par rapport au poids de la zéolithe initiale d'une couche de silice amorphe sur la surface externe de chacun des cristaux de la zéolithe EU-1.
La zéolithe EU-1 ainsi modifiée est ensuite mise en forme par extrusion (diamètre d'extrusion = 1,4 mm) avec un gel d'alumine de manière à obtenir, après séchage et calcination sous air sec, un support S2 qui contient 10 % poids de zéolithe EU-1 modifiée par CLD sous forme hydrogène et 90 % poids d'alumine.
Ce support S2 est soumis à un échange anionique avec de l'acide hexachloroplatinique en présence d'un agent compétiteur (acide chlorhydrique), de manière à déposer sur l'alumine 1 % poids de platine par rapport au poids du catalyseur. Le solide humide est ensuite séché à 120°C pendant 12 heures et calciné sous un débit d'air sec à la température de 500°C pendant une heure.
Le catalyseur B ainsi obtenu contient, en poids, 10 % de zéolithe EU-1 modifiée par CLD, 89 % d'alumine et 1 % de platine.

### Exemple 3 : Évaluation des propriétés catalytiques des catalyseurs A et B en isomérisation de l'ethylbenzène.

La charge à isomériser, mise en contact avec le catalyseur A puis avec le catalyseur B, est constituée uniquement d'éthylbenzène.

Les conditions opératoires de la réaction d'isomérisation sont les suivantes :
- température : 385°C ;
- pression totale : 10 bar (1 bar = 0,1 MPa) ;
- pression partielle d'hydrogène : 8 bar.
- charge : éthylbenzène
- vitesse spatiale d'alimentation, exprimée en kilogramme de charge introduite par kilogramme de catalyseur et par heure, égale à 4 h⁻¹.

On évalue successivement les propriétés catalytiques des catalyseurs A et B pour l'isomérisation de l'ethylbenzène. Chacun des catalyseurs A et B est réduit sous hydrogène pendant 4 heures à 480°C avant injection de la charge.

Les catalyseurs ont été évalués en terme d'activité laquelle correspond à la conversion de l'éthylbenzène.
La conversion de l'éthylbenzène notée CV_{EB} est égale à : 100 - % massique d'éthylbenzène dans l'effluent. Le pourcentage massique de l'éthylbenzène dans l'effluent est obtenu par analyse chromatographique de l'effluent.

**Tableau 1 : activité des catalyseurs A et B.**

| | Catalyseur A | Catalyseur B |
|---|---|---|
| Conversion éthylbenzène (% pds) | 38 | 33 |

Les résultats présentés dans le tableau 1 montrent que le catalyseur A comprenant une zéolithe EU-1 modifiée en surface par le dépôt d'une couche de silice amorphe via un traitement en phase gazeuse conduit à de bien meilleures performances catalytiques en terme d'activité que celles obtenues au moyen du catalyseur B comprenant une zéolithe EU-1 modifiée par une étape de traitement réalisée en présence d'un composé moléculaire contenant du silicium en phase liquide.

## Revendications

1. Procédé d'isomérisation d'une charge comprenant au moins un composé aromatique à huit atomes de carbone par molécule réalisé en présence d'au moins un catalyseur comprenant au moins une zéolithe modifiée de type structural EUO, au moins un liant et au moins un métal du groupe VIII de la classification périodique des éléments, ledit catalyseur ayant été préparé selon un procédé comportant successivement au moins :
a) une étape de traitement d'une zéolithe de type structural EUO en présence d'au moins un composé moléculaire contenant au moins un atome de silicium, au cours de laquelle ledit composé, présentant un diamètre supérieur au diamètre d'ouverture maximal des pores de ladite zéolithe, est déposé sur la surface externe de ladite zéolithe en phase gazeuse,
b) au moins une étape de traitement thermique,
c) la mise en forme de ladite zéolithe avec un liant,
d) au moins une étape d'introduction d'au moins un métal du groupe VIII de la classification périodique des éléments sur un support à base de ladite zéolithe modifiée et mise en forme.

2. Procédé d'isomérisation selon la revendication 1 tel que ledit métal du groupe VIII compris dans ledit catalyseur est le platine.

3. Procédé d'isomérisation selon la revendication 1 ou la revendication 2 tel que ledit catalyseur comprend au moins un métal additionnel choisi dans le groupe formé par les éléments des groupes IIIA, IVA et VIIB.

4. Procédé d'isomérisation selon la revendication 3 tel que ledit métal additionnel est choisi parmi l'indium, l'étain et le rhénium.

5. Procédé d'isomérisation selon l'une des revendications 1 à 4 tel que ledit catalyseur se présente sous forme d'extrudés.

6. Procédé d'isomérisation selon l'une des revendications 1 à 5 tel que ladite zéolithe de type structural EUO contient, avant d'être modifiée selon ladite étape a), au moins du silicium et de l'aluminium dans une proportion telle que le rapport atomique Si/AI est compris entre 2:1 et 100:1.

7. Procédé d'isomérisation selon l'une des revendications 1 à 6 tel que ladite zéolithe de type structural EUO est la zéolithe EU-1.

8. Procédé d'isomérisation selon l'une des revendications 1 à 7 tel que ledit composé moléculaire contenant au moins un atome de silicium et employé dans ladite étape a) est choisi parmi les composés de formule Si-R₄ et Si₂-R₆ où R peut être soit de l'hydrogène, soit un groupe alkyle, aryle ou acyle, soit un groupe alkoxy (O-R'), soit un groupe hydroxyl (-OH) soit encore un halogène, R étant identique ou différent.

9. Procédé d'isomérisation selon l'une des revendications 1 à 8 tel que ledit composé moléculaire employé dans ladite étape a) présente une composition de formule générale Si-(OR')₄ où R' est un groupement alkyle, aryle ou acyle.

10. Procédé d'isomérisation selon l'une des revendications 1 à 9 tel que ladite étape a) est réalisée dans un réacteur en lit fixe.

11. Procédé d'isomérisation selon l'une des revendications 1 à 10 tel que le traitement thermique de ladite étape b) est réalisé à une température comprise entre 200 et 700°C.

12. Procédé d'isomérisation selon l'une des revendications 1 à 11 tel qu'il est mis en oeuvre selon les conditions opératoires suivantes : une température comprise entre 300°C et 500°C, une pression partielle d'hydrogène comprise entre 0,3 et 1,5 MPa, une pression totale comprise entre 0,45 et 1,9 MPa, une vitesse spatiale d'alimentation, exprimée en kilogramme de charge introduite par kilogramme de catalyseur et par heure, comprise entre 0,25 et 30 h⁻¹.

## Claims

1. A process for isomerising a feed comprising at least one aromatic compound containing eight carbon atoms per molecule carried out in the presence of at least one catalyst comprising at least one modified zeolite with structure type EUO, at least one binder and at least one metal from group VIII of the periodic table, said catalyst having been prepared using a process which comprises at least the following in succession:
a) a step for treatment of a zeolite with structure type EUO in the presence of at least one molecular compound containing at least one silicon atom, during which said compound, with a diameter greater than the maximum pore opening diameter in said zeolite, is deposited in the gas phase on the outer surface of said zeolite;
b) at least one heat treatment step;
c) forming said zeolite with a binder;
d) at least one step for introducing at least one metal from group VIII of the periodic table onto a support based on said modified and formed zeolite.

2. An isomerization process according to claim 1, in which said group VIII metal included in said catalyst is platinum.

3. An isomerization process according to claim 1 or claim 2, in which said catalyst comprises at least one additional metal selected from the group formed by elements from groups IIIA, IVA and VIIB.

4. An isomerization process according to claim 3, in which said additional metal is selected from indium, tin and rhenium.

5. An isomerization process according to one of claims 1 to 4, in which said catalyst is in the form of extrudates.

6. An isomerization process according to one of claims 1 to 5, in which before being modified in said step a), said zeolite with structure type EUO contains at least silicon and aluminium in a proportion such that the atomic ratio Si/A1 is in the range 2:1 to 100:1.

7. An isomerization process according to one of claims 1 to 6, in which said zeolite with structure type EUO is EU-1 zeolite.

8. An isomerization process according to one of claims 1 to 7, in which said molecular compound containing at least one silicon atom used in said step a) is selected from compounds with formula Si-R₄ and Si₂-R₆, where R may either be hydrogen, an alkyl, aryl or acyl group, an alkoxy group (O-R'), a hydroxyl group (-OH) or a halogen, R being identical or different.

9. An isomerization process according to one of claims 1 to 8, in which said molecular compound employed in said step a) has a composition with general formula Si-(OR')₄, where R' is an alkyl, aryl or acyl group.

10. An isomerization process according to one of claims 1 to 9, in which said step a) is carried out in a fixed bed reactor.

11. An isomerization process according to one of claims 1 to 10, in which the heat treatment of said step b) is carried out at a temperature in the range 200°C to 700°C.

12. An isomerization process according to one of claims 1 to 11, which is carried out under the following operating conditions: a temperature in the range 300°C to 500°C, a partial pressure of hydrogen in the range 0.3 to 1.5 MPa, a total pressure in the range 0.45 to 1.9 MPa, and a space velocity, expressed in kilograms of feed introduced per kilogram of catalyst per hour, in the range 0.25 to 30 h⁻¹.

## Patentansprüche

1. Verfahren zur Isomerisation einer Beschickung, die mindestens eine aromatische Verbindung mit acht Kohlenstoffatomen je Molekül umfasst, das in Gegenwart mindestens eines Katalysators ausgeführt wird, der mindestens einen modifizierten Zeolithen vom Strukturtyp EUO, mindestens ein Bindemittel und mindestens ein Metall der Gruppe VIII des Periodensystems der Elemente umfasst, wobei der Katalysator gemäß einem Verfahren hergestellt wurde, das nacheinander mindestens umfasst:
a) einen Schritt der Behandlung eines Zeolithen vom Strukturtyp EUO in Gegenwart mindestens einer Molekularverbindung, die mindestens ein Siliciumatom enthält, in deren Verlauf die Verbindung, die einen Durchmesser aufweist, der größer ist als der maximale Öffnungsdurchmesser der Poren des Zeolithen, auf der Außenfläche des Zeolithen in Gasphase abgeschieden wird,
b) mindestens einen Schritt der thermischen Behandlung,
c) die Formgebung des Zeolithen mit einem Bindemittel,
d) mindestens einen Schritt der Einführung mindestens eines Metalls der Gruppe VIII des Periodensystems der Elemente auf einem Träger auf der Basis des modifizierten und geformten Zeolithen.

2. Verfahren zur Isomerisation nach Anspruch 1, wobei das Metall der Gruppe VIII, das im Katalysator enthalten ist, Platin ist.

3. Verfahren zur Isomerisation nach Anspruch 1 oder Anspruch 2, wobei der Katalysator mindestens ein zusätzliches Metall umfasst, das aus der Gruppe, gebildet aus den Elementen der Gruppen IIIA, IVA und VIIB, ausgewählt ist.

4. Verfahren zur Isomerisation nach Anspruch 3, wobei das zusätzliche Metall aus Indium, Zinn und Rhenium ausgewählt ist.

5. Verfahren zur Isomerisation nach einem der Ansprüche 1 bis 4, wobei der Katalysator die Form von Extrudaten aufweist.

6. Verfahren zur Isomerisation nach einem der Ansprüche 1 bis 5, wobei der Zeolith vom Strukturtyp EUO, bevor er gemäß Schritt a) modifiziert wird, Silicium und Aluminium mindestens in einem Anteil enthält, dass das Si/AI-Atomverhältnis im Bereich zwischen 2:1 und 100:1 liegt.

7. Verfahren zur Isomerisation nach einem der Ansprüche 1 bis 6, wobei der Zeolith vom Strukturtyp EUO der Zeolith EU-1 ist.

8. Verfahren zur Isomerisation nach einem der Ansprüche 1 bis 7, wobei die Molekülverbindung, die mindestens ein Siliciumatom enthält und im Schritt a) benutzt wird, aus den Verbindungen der Formel Si-R₄ und Si₂-R₆ ausgewählt ist, wobei R entweder Wasserstoff oder eine Alkyl-, Aryl- oder Acylgruppe oder eine Alkoxygruppe (O-R') oder eine Hydroxylgruppe (-OH) oder auch ein Halogen sein kann, wobei R gleich oder verschieden ist.

9. Verfahren zur Isomerisation nach einem der Ansprüche 1 bis 8, wobei die in Schritt a) benutzte Molekülverbindung eine Zusammensetzung mit der allgemeinen Formel Si-(OR')₄ aufweist, wobei R' eine Alkyl-, Aryl- oder Acylgruppe ist.

10. Verfahren zur Isomerisation nach einem der Ansprüche 1 bis 9, wobei Schritt a) in einem Festbettreaktor ausgeführt wird.

11. Verfahren zur Isomerisation nach einem der Ansprüche 1 bis 10, wobei die thermische Behandlung des Schritts b) bei einer Temperatur im Bereich zwischen 200 und 700 °C ausgeführt wird.

12. Verfahren zur Isomerisation nach einem der Ansprüche 1 bis 11, wobei es gemäß der folgenden Betriebsbedingungen durchgeführt wird: Einer Temperatur im Bereich zwischen 300 °C und 500 °C, einem Wasserstoffpartialdruck im Bereich zwischen 0,3 und 1,5 MPa, einem Gesamtdruck im Bereich zwischen 0,45 und 1,9 MPa, einer Zufuhrraumgeschwindigkeit, ausgedrückt in Kilogramm der eingeführten Beschickung pro Kilogramm Katalysator und pro Stunde, im Bereich zwischen 0,25 und 30 h⁻¹.
